# EUROPÄISCHE PATENTANMELDUNG

(11) **EP 0 700 925 A1**
(43) Veröffentlichungstag der Anmeldung: **13.03.1996**
(21) Anmeldenummer: 95110921.4
(22) Anmeldetag: 12.07.1995
(51) Int. Cl.: C07H 15/04

(54) **Verfahren zur Herstellung von Alkylpolyglycosiden**

(30) Priorität: 07.09.1994 DE 4431858
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Erfindung betrifft ein Verfahren zur einstufigen Herstellung von Alkylpolyglycosiden aus Sacchariden und Alkoholen mit 8 bis 20 C-Atomen durch säurekatalysierte Glycosidierung. Dabei wird die Reaktion mit Hilfe eines Rohrreaktors unter Gleichstrombedingungen durchgeführt.

## Beschreibung

Die Erfindung betrifft ein einstufiges Verfahren zur Herstellung von Alkylpolyglycosiden durch säurekatalysierte Umsetzung von Sacchariden und Alkoholen mit 8 bis 20 C-Atomen.

Alkylpolyglycoside mit C₈- bis C₂₀-Alkylresten können ganz oder teilweise aus nachwachsenden Rohstoffen hergestellt werden. Die Alkylpolyglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkylpolyglycoside in transparenten, farblich schönen wäßrigen Lösungen hergestellt werden können.

Zur Herstellung von Alkylpolyglycosiden mit langkettigen Alkylgruppen kann man zunächst durch Glycosidierung von Sacchariden mit kurzkettigen Alkoholen Alkylglycoside mit C₁- bis C₆-Alkylgruppen herstellen. Diese Produkte werden dann mit langkettigen Alkoholen durch Umglycosidierung bei erhöhter Temperatur in die gewünschten Alkylpolyglycoside übergeführt. Die so hergestellten Produkte sind jedoch dunkel gefärbt.

Unter Einhaltung bestimmter Mengenverhältnisse und bei Verwendung von Löslichkeitsvermittlern können die polaren Saccharide auch direkt mit den unpolaren langkettigen Alkoholen zu den Alkylpolyglycosiden umgesetzt werden. Dabei werden auch hier ohne Zusatz von farbverbessernden Mitteln, wenn die Reaktion im Rührkessel durchgeführt wird, dunkelfarbige Produkte erhalten.

In EP 0 077 167 wird ein einstufiges Herstellverfahren beschrieben, bei dem eine Aldose oder eine Ketose direkt mit einem langkettigen Alkohol im Molverhältnis von 1 : 1,25 bis 1 : 4 umgesetzt wird. Die Reaktion wird bei geringen Wassergehalten in Gegenwart eines Reduktionsmittels durchgeführt.

Dieses einstufige Verfahren wird in DE-A-41 01 252 dadurch verbessert, daß man einen großen Alkoholüberschuß verwendet und zur Neutralisation in Alkoholen gelöstes Alkalihydroxid verwendet. Die Reaktion wird hier in einem Rührkessel ausgeführt.

Darüber hinaus ist bekannt, daß man die Farbqualität der Alkylpolyglycoside durch apparative Maßnahmen verbessern kann.

So kann man nach EP-A-0 482 325 bei einem zweistufigen Herstellverfahren die 1. Stufe, die Glycosidierung von Sacchariden in wäßriger Lösung mit C₁- bis C₆-Alkoholen, in einer Gegenstromreaktionskolonne` beispielsweise in einer Glockenbodenkolonne, durchführen.

Außerdem kann nach DE-A-41 16 665 auch die Umglycosidierung, die 2. Stufe des zweistufigen Verfahrens, in einer Reaktionskolonne und vorzugsweise unter Gegenstrombedingungen ausgeführt werden.

Das zweistufige Verfahren mit zwei hintereinander geschalteten Kolonnen ist jedoch mit einem relativ hohen apparativen Aufwand verbunden.

Aufgabe der vorliegenden Erfindung war es daher, die Nachteile des Standes der Technik zu vermeiden und ohne Zusatz von Reduktionsmitteln in einem einfachen Verfahren hellfarbige Produkte herzustellen.

Die Aufgabe wird erfindungsgemäß dadurch gelöst, daß man bei einer einstufigen Herstellung von Alkylpolyglycosiden die Reaktion mit Hilfe eines Rohrreaktors unter Gleichstrombedingungen durchführt.

Geeignete Rohrreaktoren haben im allgemeinen einen Durchmesser von 0,5 bis 50 cm und eine Länge von 0,5 bis 50 m, wobei die Länge mindestens das 4fache des Durchmessers sein soll. Im einfachsten Falle handelt es sich dabei um ein beheizbares Rohr. Vorzugsweise weist der Rohrreaktor jedoch auch mehrere Stutzen auf, über die Vakuum angelegt und eingeführtes und gebildetes Wasser abgezogen werden kann.

Der Rohrreaktor kann auch Schikanen und Verwirbelungseinbauten enthalten. Das Rohr kann horizontal, schräg oder vertikal angeordnet sein, so daß im Sinne dieser Erfindung auch Kolonnen, beispielsweise Glockenbodenkolonnen, als Rohrreaktoren angesehen werden. Dabei ist allerdings darauf zu achten, daß die Reaktionskomponenten im Gleichstrom geführt werden.

Als Saccharide können Aldosen und Ketosen eingesetzt werden. Beispiele dafür sind Glucose, Mannose, Galaktose und Fructose. Dabei wird Glucose vorzugsweise verwendet. Die Saccharide können wasserfrei oder als wäßriger Sirup eingesetzt werden.

Für das vorliegende Verfahren geeignete Alkohole sind beispielsweise Octanol, Decanol, Laurylalkohol, Myristyl-, Palmityl- und Stearylalkohol. Es können auch Gemische von Alkoholen eingesetzt werden. Vorzugsweise verwendet man Alkohole mit 8 bis 12 C-Atomen.

Das Saccharid/Alkohol-Molverhältnis liegt bevorzugt im Bereich von 1 : 2 bis 1 : 10.

Als Katalysatoren sind Mineralsäuren und starke organische Säuren geeignet. Beispiele dafür sind Schwefelsäure, Phosphorsäure und p-Toluolsulfonsäure. Der Katalysator wird vorzugsweise in Konzentrationen von 0,2 bis 5 %, bezogen auf das Saccharid, eingesetzt.

Die Reaktion wird meist bei einer Temperatur von 50 bis 140 °C durchgeführt. Dabei werden Temperaturen von 70 bis 130 °C besonders bevorzugt.

Gleichzeitig wird vorzugsweise eine mittlere Verweilzeit von 5 bis 90 Minuten eingestellt.

Das Verfahren wird vorzugsweise kontinuierlich durchgeführt. Die Produkte weisen im allgemeinen einen mittleren Glycosidierungsgrad von 1 bis 10 auf, wobei mittlere Glycosidierungsgrade von 1,1 bis 4 besonders bevorzugt und mittlere Glycosidierungsgrade von 1,1 bis 1,4 ganz besonders bevorzugt eingestellt werden.

Durch die vorliegende Erfindung wird insbesondere bei kontinuierlich arbeitenden Syntheseanlagen der apparative Aufwand stark vermindert. Durch die kurzen Verweilzeiten und die engen Verweilzeitverteilungen erhält man wegen der geringen thermischen Belastung helle Produkte von hoher Qualität. Hellfarbig sind die Produkte, wenn ihre 50%igen wäßrigen Lösungen vor der H₂O₂-Bleichung Jodfarbzahlen von < 60 aufweisen.

Nach der Reaktion wird das Produkt in bekannter Art und Weise mit einer Base neutralisiert, worauf überschüssiger Fettalkohol destillativ abgetrennt wird. Anschließend wird das Produkt im allgemeinen mit Wasser abgemischt und mit H₂O₂ gebleicht.

Bei der praktischen Durchführung der Erfindung können dem Rohrreaktor auch Rührkessel vor- oder nachgeschaltet sein. So kann man beispielsweise dann, wenn man Sirup einsetzt, Wasser unter leichtem Vakuum bei 70 bis 130 °C im Rührkessel entfernen, bevor man Katalysator zusetzt und die Mischung in den Rohrreaktor leitet. Man kann das Wasser auch mit Hilfe von Inertgas strippen. Man kann den Katalysator auch bereits in einen vorgeschalteten Rührkessel geben, so daß dann neben der Entwässerung die Verbindungen zu einem Teil reagieren. Das anreagierte Reaktionsgemisch wird dann vorzugsweise über einen Mischer homogenisiert und in den Rohrreaktor geleitet. Nachgeschaltete Rührreaktoren dienen im allgemeinen nur dazu, die Verweilzeit zu erhöhen und den Umsatz zu vervollständigen. Vorzugsweise werden 5 bis 80 %, in besonderen Fällen 10 bis 60 %, der Reaktion in einem Rohrreaktor durchgeführt.

### Beispiel

Ein 25-l-Rührreaktor wird stündlich mit einem Gemisch aus 14,7 kg Fettalkohol (66 % Dodecanol, 28 % Tetradecanol, 6 % Hexadecanol) und 1,8 kg wasserfreier Glucose gespeist. Durch Zudosieren von p-Toluolsulfonsäure wird im Reaktor eine Katalysatorkonzentration von 0,25 Gewichtsprozent aufrechterhalten. Die mittlere Verweilzeit beträgt 30 Minuten. Bei einer Innentemperatur von 110 °C wird ein Vakuum von 30 mbar angelegt, so daß Reaktionswasser zügig abdestilliert werden kann. Der Reaktoraustrag weist noch einen Glucosegehalt von 2,1 % auf.

Das Reaktionsgemisch wird durch einen statischen Mischer und dann durch ein beheizbares Rohr mit einem Innendurchmesser von 10 cm und einer Länge von 175 cm geleitet. Das Reaktionsgemisch enthält 77,6 % Fettalkohol, 12,9 % Alkylglucosid mit einem mittleren Glycosidierungsgrad von 1,26 und < 0,1 % Glucose.

## Patentansprüche

1. Verfahren zur Herstellung von Alkylpolyglycosiden aus Sacchariden und Alkoholen mit 8 bis 20 C-Atomen durch säurekatalysierte Glycosidierung,
dadurch gekennzeichnet,
daß man die Reaktion mit Hilfe eines Rohrreaktors unter Gleichstrombedingungen durchführt.

2. Verfahren nach Anspruch 1,
dadurch gekennzeichnet,
daß man die Reaktion bei 70 bis 130 °C durchführt.

3. Verfahren nach Anspruch 1,
dadurch gekennzeichnet,
daß man bei der Reaktion eine mittlere Verweilzeit von 5 bis 90 Minuten einstellt.

4. Verfahren nach Anspruch 1,
dadurch gekennzeichnet,
daß man Alkohole mit 8 bis 12 C-Atomen umsetzt.

5. Verfahren nach Anspruch 1,
dadurch gekennzeichnet,
daß man 5 bis 80 % der Reaktion in einem Rohrreaktor ausführt.
